# EUROPEAN PATENT APPLICATION

(11) **EP 4 537 779 A1**
(43) Date of publication of application: **16.04.2025**
(21) Application number: 23202667.4
(22) Date of filing: 10.10.2023
(51) Int. Cl.: A61B 18/20, A45D 26/00

(54) **THE METHOD OF SELECTING THE PARAMETERS FOR SETTING THE LASER HAIR REMOVAL HEAD BASED ON SKIN AND HAIR COLOUR, THE DEVICE AND THE HAIR REMOVAL SYSTEM IMPLEMENTING THIS METHOD**

(71) Applicant: DEPILACJA.PL SPOLKA Z O. O., 02 715 Warsaw (PL)
(72) Inventor: Jankowski, Bartlomiej, 01 315 Warsaw (PL); Kalinowski, Maciej, 05 532 Chojnow (PL); Banacki, Marcin, 05 220 Zielonka (PL); Wasylewski, Marcin, 04 634 Warsaw (PL); Korzekwa, Adrian, 04 041 Warsaw (PL); Poleszczuk, Piotr, 20 826 Lublin (PL); Furmanski, Piotr, 05 270 Marki (PL); Grzeszczyk, Michal, 02 612 Warsaw (PL); Kochanowski, Jakub, 11 036 Gietrzwald (PL); Zatka, Nina, 87 500 Rypin (PL); Kucharski, Tomasz, 02 652 Warsaw (PL); Tymczyna, Pawel, 02 972 Warsaw (PL); Mikolajczyk, Piotr, 01 107 Warsaw (PL); Tymczyna Sobotka, Monika, 20 868 Lublin (PL)
(74) Representative: Kondrat, Mariusz

(57) **Abstract**

The subject of the invention is a method for selecting the setting parameters of a laser hair removal head (3) based on skin and hair colour, characterised in that it comprises the following steps : a) transmission of the request to measure the patient's skin and hair parameters to the central unit 11; b) Initiation by the central unit (11) of the measurement of the patient's body temperature by sending a request to the HSI handle controller (19), which initiates the temperature measurement by means of the contactless temperature sensor (28) of the HSI handle (4); c) Initiation by the central processing unit (11) of taking a series of images with the HSI camera (20) by sending a request to the HSI handle controller (19), where on each command issued by the central processing unit (11), the HSI handle controller (19) initiates the activation of at least one LED from the board (24) to illuminate the patient work area with the selected wavelength and to take an image with the HSI camera (20), and each of the images taken in the series in real time is evaluated by the central processing unit (11) for a focus and offset criterion; d) Storing each of the images taken in the memory of the central unit (11) along with metadata including temperature and body humidity; f) Transfer of stored images with metadata as input to the neural network implemented in the CPU memory (11); g) Classification by neural network of the patient's skin and hair colour; h)Transmission of the classification determined in step f) to the reinforcement learning network (reinforcement learning), which, on the basis of input data, generates the optimum setting for the epilation head (3) in the form of a parameter vector containing for each wavelength: laser intensity levels, pulse duration per period, operating frequency, length of time the machine operates at the given parameters, the said input data being at least the classification data from step (f).

## Description

The object of the invention is a method for selecting the setting parameters (configuration) of a laser hair removal head based on skin and hair colour, a device and a hair removal system implementing this method.

The current standard for selecting parameters for laser hair removal treatments is a visual assessment of skin and hair colour by a cosmetologist. Prior to the first visit, patients are asked to shave the area to be treated and leave a few hairs - this allows for a simpler assessment than with fully shaved skin. Based on the remaining hair and skin colour, the cosmetologist decides on the parameters of the laser epilator (power, power per unit area and treatment duration). Sometimes, existing allergies, the patient's perceived pain threshold and other factors that may affect the effectiveness of the procedure are additionally analysed. The cosmetologist's assessment of the skin and hair is therefore superficial, and the parameters chosen for the treatment are very often similar. Cosmetologists are guided by well-known methods that, although they work, do not provide optimal treatment results tailored to the individual patient. Unfortunately, such a procedure is prone to human error and inadequate assessment of the patient's skin condition, which can lead to ineffective treatment (hair reduction of less than 10%) or, in the worst case scenario, burns and skin damage.

From the state of the art, ways of detecting skin using light and thermal sensors are known. For example, document WO2016116307 A1 discloses a device for detecting skin areas within an analysed surface, which includes a thermal sensor input acquiring data from a thermal sensor of the scene, a light sensor input acquiring data from a light sensor and an evaluation unit for analysing the acquired data. Preferably, said thermal sensor and said light sensor are arranged to have essentially the same optical path. Whereby said evaluation unit is configured to select the first spatial areas within the thermal sensor data with data values within a predetermined range (in particular between 30 and 42°C), to select the second spatial areas within the light sensor data with data values within a predetermined range, to select the third and/or fourth spatial areas by determining the photoplethysmography, PPG signals from the light sensor data and to select the third and/or fourth spatial area within the light sensor data with the highest PPG signal strength and/or with the strongest pulsation and/or to select the fifth spatial areas within the light sensor data with the highest spatial homogeneity of chrominance values and/or intensity of adjacent pixels, and to correlate said first and second spatial areas with one or more of said third, fourth and fifth spatial areas to detect skin areas. Preferably, the aforementioned thermal sensor includes a long-wavelength camera unit for acquiring thermal images in the long-wavelength infrared spectrum. Preferably, said light sensor comprises an imaging unit for acquiring images in the visible light and/or infrared spectrum. The document also discloses a method of detecting skin in a test area comprising obtaining thermal sensor data of the scene; obtaining light sensor data of the scene; analysing the obtained thermal sensor data and the obtained light sensor data; selecting first spatial areas within the thermal sensor data with data values within a predetermined range; selecting the second spatial areas within the light sensor data with data values within a predetermined range, and selecting the third and/or fourth spatial areas by determining the photoplethysmography, PPG, signals from the light sensor data and selecting the third and/or fourth spatial areas within the light sensor data with the highest PPG signal strength and/or with the strongest pulsation and/or to select the fifth spatial areas within the light sensor data with the highest spatial uniformity of chrominance values and/or intensity of adjacent pixels, and detecting skin areas within the scene based on said analysis by correlating said first and second spatial areas with one or more of said third, fourth and fifth spatial areas.

In addition, there are known systems and ways to assess skin types or hair colour using digital imaging. Document US2020226660 relates to a digital imaging method of analysing pixelated digital images of the face to determine the user's face shape and ability to grow hair, and recommending a hairstyle based on face type and hair growth ability. The disclosed method includes: a. acquiring a digital facial image of a user using a digital camera; b. collecting digital facial images from a plurality of individuals from a database; c. using a neural network to compare a plurality of pixels from a digital facial image of a user with a plurality of pixels from digital facial images of a plurality of individuals to determine a shape type of the user's face; d. using a neural network to compare a plurality of pixels from a digital facial image of a user with a plurality of pixels forming digital facial images of a plurality of individuals to determine a user's ability to grow hair in an area of the user's face; and e. recommending to the user via interface logic in the computing device a facial hairstyle based on the user's face shape type and the user's ability to grow hair in the user's facial area. Preferably, the method additionally comprises the steps of: f. selecting, using analysis logic in the computing device, a product from at least two available products for the user to obtain a recommended beard style based on the user's face type and the user's ability to grow hair in the user's facial region; and g. recommending, using interface logic in the computing device, the selected product to the user.

From document US2012300050, a method of characterising skin tone is known which includes: capturing a digital colour image of at least one skin zone containing a plurality of N pixels, each pixel having a grey level; extracting three colour planes from said colour image named Red (R), Green (G) and Blue (B); evaluating the skin colour or coatings based on the grey level value of each pixel from at least some N pixels of the image for each colour plane of at least two colour planes out of the three colour planes R, G, B; an assessment of the Luminance L* based on the grey level value of each pixel from at least some N image pixels for each colour plane of at least two colour planes out of the three colour planes R, G, B; and a characterisation of the skin tone or coatings resulting from both the said skin tone or coatings assessment and the said Luminance L* assessment.

Document US20060149151 discloses a cosmetic colour determination system comprising a light collector with significant sensitivity above 700 nm (preferably above 800 nm); and an analytical component that uses infrared data from the light collector to determine cosmetic hair or skin colour. Preferably, the light collector also has significant sensitivity in the range of visible red and green wavelengths, and the analytical component uses the visible wavelengths to determine hair colour. In addition, the aforementioned collector can also have significant sensitivity in the range of visible wavelengths of red, green and blue, and the analytical component uses visible wavelengths to determine hair colour. In addition, the system includes a mechanism to outweigh individual wavelengths or wavelength ranges when determining hair colour. In addition, the document discloses a product recommendation system comprising said colour determination system, a database of hair dye products and software that uses information from the cosmetic colour determination system to select a hair dye product from the database. Furthermore, the system for determining skin colour, includes a hand-held adaptor that carries a light collector, and which is sized and dimensioned for use on facial skin or mucous membranes.

From WO0123850 A1, there is known a handheld scanner for determining the colour of a body element, comprising: a housing containing a light source and a detector containing a photodiode; a first guiding element which is coupled to the housing and in contact with the body element and which guides the housing in a substantially linear motion relative to the body element; and a second guiding element which is coupled to the housing and records the movement of the scanner relative to the body element. The housing may possibly include a handle. The light source is an incandescent light source, preferably a fluorescent light source. The scanner is provided with a central unit electronically connected to the scanner via a signal transmission part; and an automated mixing device electronically connected to the central processing unit, the automated mixing device mixes at least two ingredients to produce a cosmetic composition under the control of the central processing unit. Preferably, the cosmetic composition is selected from the group consisting of a hair dye solution, a tanning solution, a cosmetic foundation and a tattoo ink. Further, the document discloses a method of matching the colour of the body component with the colour of the cosmetic composition, which includes sourcing the colour composition of the body component at multiple locations; obtaining a dominant colour shade from the colour composition using a central processing unit; displaying multiple colours selected from different shades of the dominant colour shade; visually selecting an individual colour from the multiple colours; and a central processing unit controlling a mixing device that combines multiple cosmetic components to produce a cosmetic composition that matches the selected individual colour.

Document US20060149151 discloses a cosmetic colour determination system comprising a light collector with significant sensitivity above 700 nm (preferably above 800 nm); and an analytical component that uses infrared data from the light collector to determine cosmetic hair or skin colour. Preferably, the light collector also has significant sensitivity in the range of visible red and green wavelengths, and the analytical component uses the visible wavelengths to determine hair colour. In addition, the aforementioned collector can also have significant sensitivity in the range of visible wavelengths of red, green and blue, and the analytical component uses visible wavelengths to determine hair colour. In addition, the system includes a mechanism to outweigh individual wavelengths or wavelength ranges when determining hair colour. In addition, the document discloses a product recommendation system comprising said colour determination system, a database of hair dye products and software that uses information from the cosmetic colour determination system to select a hair dye product from the database. Furthermore, the system for determining skin colour, includes a hand-held adaptor that carries a light collector, and which is sized and dimensioned for use on facial skin or mucous membranes.

A portable skin diagnostic device comprising: a first measurement system and a second measurement system is known from EP2339964. Whereby, the first measurement system comprises a diffuser; a retrieval aperture to be placed in front of the skin surface; an illumination system to illuminate the skin in the retrieval aperture; an optical detector that receives the light reflected by the skin placed in the retrieval aperture. Whereby, the illumination system is positioned behind the diffuser so as to illuminate the skin at the retrieval aperture through the diffuser; and the optical detector is positioned in an opaque tube that faces the retrieval aperture, the illumination system surrounds the opaque tube and a diffuser containing a material that is transparent to the wavelengths of light emitted by the illumination system, and the diffuser presents a shape that is concave towards the retrieval aperture. In contrast, the second measurement system is configured to take measurements on the skin in a non-optical manner. The disclosed lighting system contains multiple LEDs that emit light at different wavelengths. Preferably, the lighting system comprises one or more IR LEDs or one or more LEDs or UV LEDs emitting at red, green and blue wavelengths, respectively.

The purpose of the invention is to provide a new self-learning system and a method to manage a network of self-learning hair removal devices.

The essence of the invention is a method for selecting laser hair removal head setting parameters based on skin and hair colour, characterised in that it comprises the following steps:
a) sending a request to measure the patient's skin and hair parameters to the central unit;
b) initiation by the central unit of the measurement of the patient's body temperature by sending a request to the HSI handle controller, which initiates the temperature measurement using the contactless temperature sensor of the HSI handle;
c) initiation by the CPU of taking a series of images with the HSI camera by sending a request to the HSI handle controller, where on each command issued by the CPU, the HSI handle controller initiates the activation of at least one LED from the board to illuminate the patient work area with the selected wavelength and to take an image with the HSI camera, and each of the images taken in the series in real time is evaluated by the CPU for a focus and offset criterion;
d) storing each of the images taken in the central unit's memory along with metadata including body temperature and humidity;
e) sending the stored images with metadata as input to the neural network implemented in the central unit's memory;
f) classification by neural network of the patient's skin and hair colour;
g) transmission of the classification determined in step f) to a reinforced learning network which, on the basis of the input data, generates the optimal setting for the epilation head (3) in the form of a vector of parameters containing for each wavelength: laser intensity levels, pulse duration per period, operating frequency, length of operation of the machine at the given parameters, the said input data being at least the classification data from step f).

Preferably, in step (b), wavelengths in the range 370 nm to 1050 nm are used to take a series of images by the HSI camera.

Preferably the series of images taken in step c) are at least 3 images, preferably 5 images, 10 images, 14 images, 50 images, 150 images, 500 images.

Preferably, in the method according to the invention the step of approving the selected setting (setting) of the epilation head by presenting the selected set value (setting) to the specialist performing the epilation treatment with the option of manual adjustment.

Preferably, in step g) the input data used are data obtained from step f) in combination with at least one parameter selected from a group of metadata comprising: part of epilated body fragment , gender, age, personal data of the patient in question, information on pregnancy, information on breastfeeding, information on autoimmune diseases, data on medications taken, information on cosmetics used, information on herbal remedies taken, information on visits to a solarium within the last 4-6 weeks, date of last epilation, date of last peeling and type of peeling, tendency of the skin to hyperpigmentation, occurrence of dermatological skin lesions, occurrence of Reticularis, history of photodermatoses, history of past or ongoing viral/fungal/bacterial infections, history of epilepsy, history of diabetes mellitus, presence of pacemaker, use of insulin pump, history of scarring, HPV infection, history of eczema, difficulties with wound healing, use of steroid medication, complexion type, presence of ingrown hairs, presence of folliculitis, presence of hormonal disorders, any tattoos, any permanent make-up, any implants or combination of the above.

Preferably, the method according to the invention comprises a step of training a neural network for skin and hair colour classification and a reinforcement learning network by uploading the results obtained in steps f) and g) to a cloud server, where training instances of the network are created on the basis of the newly added data in combination with the data contained in the database and an additional evaluation of the images in terms of hair density and skin condition of the client is carried out, and the analysis results obtained are sent to the memory of the central unit in order to train the networks stored therein to perform steps f) and g).

Another embodiment of the invention is an epilation device having a body, a central unit, a display, a cooling module, power supplies and a laser epilation head, characterised in that:
is equipped with an HSI handle, which consists of a housing inside which a hygroscopic camera with lens, a diode board and the HSI handle controller are mounted, while a contactless temperature sensor is located on the bottom of the housing, below the HSI camera;
the laser epilation head is equipped with a laser diode stacks of three wavelengths and a epilation handpiece controller module for setting treatment parameters based on data from the central unit;
the central unit consists of a processor and memory, with Edge Computing architecture implemented, containing instructions for performing the selection of the setting parameters of the laser hair removal head based on skin and hair colour according to the invention.

Preferably, the HSI handle housing is equipped with an indicator LED and an information LED. Preferably, the three wavelengths of the laser diode stacks include wavelengths of 808 nm±5nm, 1064 nm±5nm and 755 nm±5nm, respectively.

Preferably, the HSI handle is housed in a lockable body pocket.

Preferably, each diode of the laser diode stack is controlled independently by a dedicated laser diode stack controller of the epilation head.

Preferably, step f ') is followed by step f'), in which the photo is segmented using a neural network of hair follicles, the number of follicles is counted and the number of hairs visible in the photo is sent to the reinforcement learning network.

A further embodiment of the invention is a networked epilation device system having at least one epilation device, a cloud server and a database, characterised in that said at least one epilation device is a device according to the invention, the database comprises skin colour data, hair colour data, photos of the skin, photos of the hair, photos of the epilation treatments carried out, classification of the skin colour and hair colour, parameters of the epilation head matched to the skin colour and hair colour, and the cloud server is configured to select the setting parameters of the laser epilation head based on the skin colour and hair colour by the method according to the invention.

The invention provides the following benefits:
- basing the action on hyperspectral imaging of the skin and hair ensures that the effectiveness and safety of the treatment is optimised;
- personalisation of the settings for the specific patient to be treated - allows the skin and hair structure of each patient to be analysed individually. In this way, the laser parameters proposed for treatment by the model are tailored to the individual user and lead to a treatment that allows maximum hair reduction with a high degree of safety;
- updating of all devices according to the invention connected to the system according to the invention by newly acquired data from subsequent epilation treatments - hyperspectral imaging of the area to be epilated is used before the treatment is carried out. During each treatment, the cosmetologist performs the assessment presented and the result of this method, together with the hyperspectral image, is transmitted to the self-learning network. This enables the network to independently analyse the structure of the patient's skin and hair, including deep within the skin - hyperspectral imaging enables the visualisation of reflected electromagnetic radiation from the visible light, ultraviolet and near-infrared ranges through the patient's skin. Images transmitted to the network show the hair follicles hidden beneath the skin and help select parameters for the treatment;
- the possibility of dynamically attaching further (new) devices according to the invention to the system according to the invention without losing the knowledge acquired on other devices, since the system aggregates the data acquired from each device during the treatments and updates its parameters (during the learning process). This is followed by the start of the update process for models located on local devices using Edge Computing. The model parameters stored in the central unit can be used to prepare a new device in such a way that a new device introduced into the practice retains all the knowledge of the other devices in the system according to the invention;
- the possibility of off-line operation of system peripherals (i.e. hair removal appliances);
- allows the patient's skin to be analysed by computer vision algorithms and the use of artificial intelligence systems to accurately assess skin and hair colour;
- the use of a self-learning approach leads to the benefit of the cosmetologists' expertise while analysing the effects of each treatment and the possibility of improving the results obtained;
- the solution according to the invention, after analysis, presents the cosmetologist with the predicted skin and hair type, and after acceptance (or possible improvement) of these metrics, an estimation of the laser parameters enabling an optimal and safe treatment is made. Moreover, at each subsequent visit, further hyperspectral images are taken and the patient is interviewed about the results achieved and any complications. All these parameters are presented to the self-learning network and, once again, have an impact on its learning;
- the whole process is supervised by a human (cosmetologist) to ensure maximum safety. Over time, the model will obtain optimal metrics that generalise to the cosmetologists' knowledge, the effects of the treatments, and the laser parameters selected by the network should produce better hair removal results than if the cosmetologist performed the treatment alone;
- the entire system according to the invention is designed in such a way that the lack of an internet network does not interfere with the operation of the hair removal devices according to the invention incorporated therein. The artificial intelligence models are stored on the central processing unit of each device. Instead of relying on a model located on a server, Edge Computing is used, which allows laser parameters to be selected through a local model without the need for a network connection. The whole system continues to work in a way that, for the cosmetologist/patient, there is no change in the quality of the treatment in the event of a network failure. When the internet connection is restored, data stored locally is synchronised with the cloud server, so that no knowledge gained is lost.
- the use of the HSI camera handle provides a low-cost solution for hyperspectral imaging, as the HSI camera used in the device according to the invention is made up of readily available commercially components, i.e. an RGB camera, illuminating diodes and electronic components used on PCBs together with a specially adapted, dedicated housing produced using, for example, 3D printing technology. Competing hyperspectral solutions, on the other hand, are an order of magnitude more expensive, and changing a component therein in subsequent versions of the product, involves significant costs, in contrast to the HSI handle of the device according to the invention, in which LEDs or photodiodes can be replaced by others, which will not significantly affect the performance of the device according to the invention. Similarly for the RGB camera and lens used in the solution according to the invention,
- due to the multitude of similar characteristics of lenses and CMOS sensors available on the market, in the event that a particular component used in the HSI handle of the device according to the invention disappears from the market, any other sensor or lens with similar characteristics can be used. Since the device according to the invention creates HSI images in a post-processing block, using illumination for many different wavelengths of the test object, with fixed camera parameters, for a small area of the selected image fragment, the HSI handle images are obtained within less than 1 second, while commercially available on the market HSI cameras usually operate using diffraction elements that mechanically change the orientation of the diffraction element using the phenomenon of spatial and temporal coherence, which determines the data acquisition length by min. an order of magnitude longer and only allows for the analysis of static obj ects;
- in contrast to known market solutions, the epilation head of the device according to the invention is distinguished by the fact that each of the wavelengths used (755 nm, 808 nm, 1064 nm) is independently controllable;
- the epilation head of the device also has a significant power reserve, which means that for different skin and hair types, different values for each wavelength can be selected for the same power, which is more optimal. The available, competing devices also use multiple wavelengths, but applying them and changing parameters is the same for all of them due to their use of a single controller. This takes away their degrees of freedom when optimising the parameters of the laser hair removal treatment.

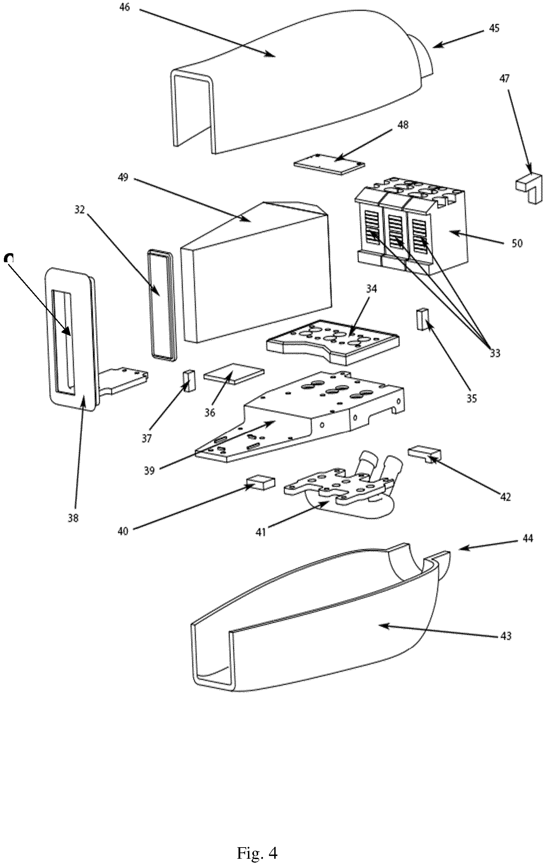

The invention is illustrated in the manufacturing example and in the drawing in which fig. 1 shows the device according to the invention in a demonstrative view; fig. 2 shows a schematic of the HSI camera handle of the device according to the invention in a demonstrative view; fig. 3 shows a block diagram of the HSI handle of the device according to the invention; fig. 4 shows a diagram of the laser hair removal head of the device according to the invention; fig. 5 shows a block diagram of the laser hair removal head of the device according to the invention; fig. 6 shows a schematic of the system according to the invention.

### Example 1.

### The device according to the invention

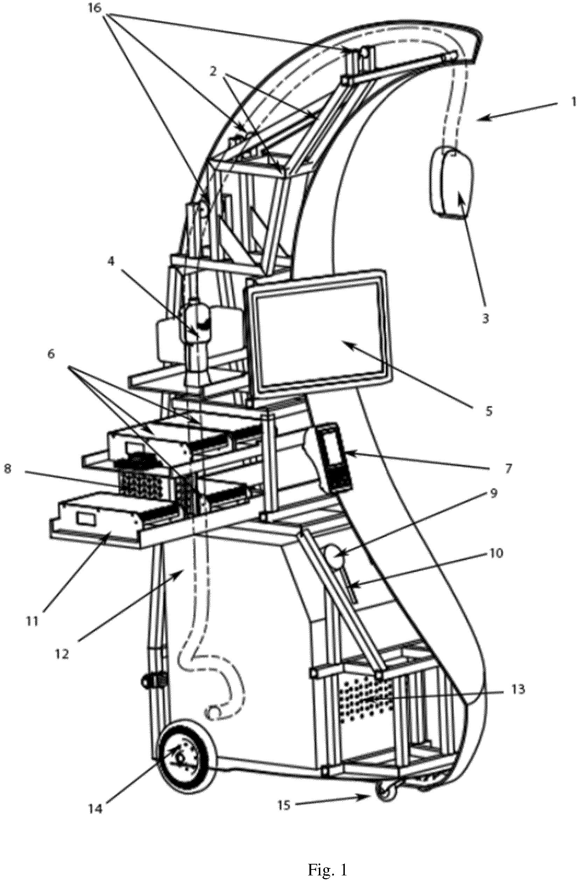

The device according to the invention is shown in fig. 1, wherein 1 indicates hydraulic line; 2 indicates body; 3 indicates laser epilation head; 4 indicates hygroscope camera handle; 5 indicates display; 6 indicates laser diode stack controllers of the epilation head; 7 indicates payment terminal; 8 indicates power supply controller 6; 9 indicates coolant inlet; 10 indicates fluid level indicator; 11 indicates central unit;
12 indicates cooling module; 13 indicates cooling module ventilation; 14 indicates non-turning wheels; 15 indicates turning wheels; 16 indicates guides.

The body 2 of the device according to the invention encloses components including a cooling module 12 with its ventilation 13 and a reservoir for the cooling liquid (e.g. distilled water); a central unit 11, a display 5, controllers 6 and 8, a hydraulic line 1.

The unit is connected to 230V mains voltage. This voltage is converted to 5V and 12V powering the rest of the device components apart from the laser diode stacks 33. These stacks are powered from separate, three power supplies that convert 230V to a regulated output in a voltage-corrected current source (power source) arrangement.

In this example of implementation, the body 2 of the device according to the invention is provided with two pairs of wheels, i.e. a rear pair of non-turning wheels 14 and a front pair of turning wheels 15. With that said, the front wheels are smaller than the rear wheels. Furthermore, in this example implementation, a payment terminal 7 is incorporated in the body 2 of the device according to the invention. In this embodiment, the device according to the invention is also provided with a wheel locking element 14 and 15.

The most important and also the main control element of the device according to the invention is the central unit 11, which supervises and controls all subordinate modules, namely the HSI handle 4, the power supply controller 8 and the cooling module 12. In turn, it indirectly manages the operation of the epilatory handle 4.

In this embodiment, the central processing unit 11 is a "Jetson Nano minicomputer - equipped with an ARM Cortex A57 Quad-Core processor clocked at 1.43 GHz and an Nvidia Maxwell GPU with 128 CUDA cores. This minicomputer has 4GB of DDR4 RAM, a Gigabit Ethernet port, HDMI and Display ports, 4x USB 3.0, GPIO, I2C, I2S, SPI, UART, 2x camera connectors and a microSD card slot." In addition, the central unit was retrofitted with a WiFi wireless communication expansion card.

In this embodiment, cooling module 12 is the NEXTREME 1200 Recirculating Chiller from Laird Thermal Systems. This part of the device performs the function of regulating and cooling the diode stacks 33 of the laser epilation head 3, ensuring that they have the proper working conditions. It prevents them from overheating, which could consequently lead to permanent damage. The cooling module 12 is also controlled from the central unit 11. An RS232 interface is used for communication. Hydraulic hoses 1 are fed from the cooling module 12 up to the spigots of the heat exchanger 39/41/34 directly adjacent to the laser diode stacks 33, creating a closed circuit for the cooling liquid in the form of distilled water.

In this embodiment, the air for cooling module 12 is drawn in through HEPA filters from the floor side.

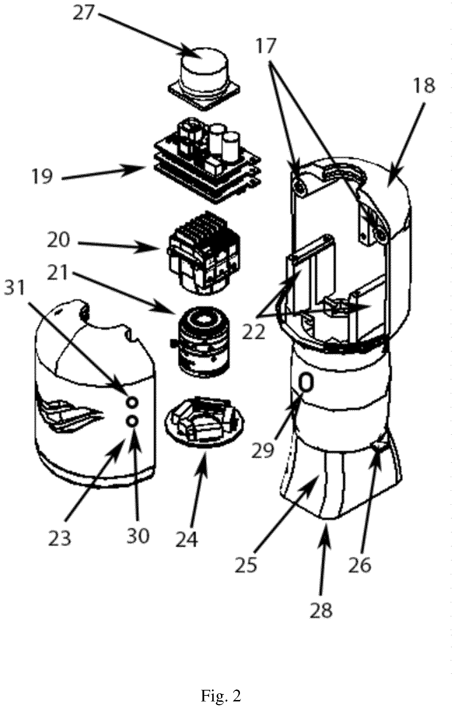

In contrast, the hygroscopic camera handle 4 (HSI handle) is shown in fig. 2, where 17 denotes the mechanical connection between the parts of the HSI handle housing; 18 denotes the HSI handle housing; 19 denotes the HSI handle controller; 20 denotes the HSI camera; 21 denotes the lens; 22 denotes the mounting location of the controller 19 in the housing 18; 23 denotes the removable part of the housing; 24 denotes diode board; 25 denotes working field cover; 26 denotes lens focus adjustment; 27 denotes plug-in connector with power and communication cables; 28 denotes contactless temperature reader; 29 denotes button; 30 denotes indicator LED; 31 denotes information LED.

In this embodiment, the HSI 4 handle consists of a lightweight, compact 20 Allied Vision Alvium 1800 U-500c camera with a Tamron (2f) 21 f=12mm, F/2.4 lens. On the other hand, communication with the central unit 11 of the device according to the invention takes place via the USB 3.1 gen. 1.

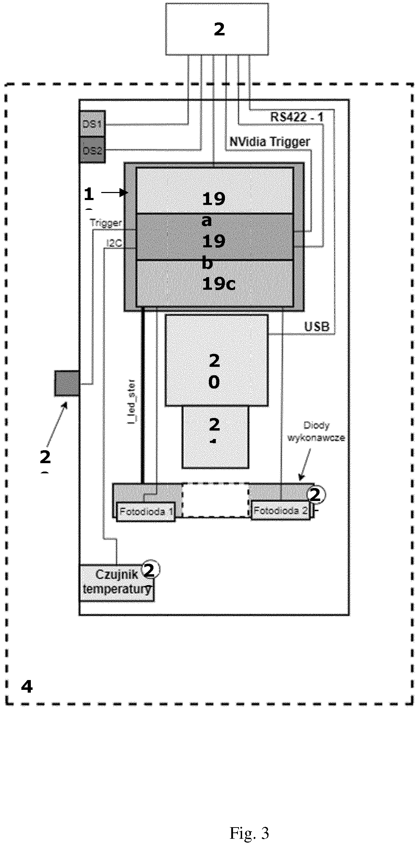

In this embodiment, the handle controller HSI 19 is an electronics module, an example of which is shown in fig. 3, where: 2 denotes body, 4 denotes HSI handle 4, 19 denotes HSI handle controller, 19a denotes HSI power module, 19b denotes HSI control module, 19c denotes HSI control module, 20 denotes HSI camera, 21 denotes lens, 24 denotes actuator diode board, 28 denotes contactless temperature sensor and 29 denotes button.

As indicated in fig. 3, in this embodiment, the HSI 19 handle controller is an electronics module communicating over an RS422 interface (in fig. 3 designated RS422-1) with central unit 11. The HSI handle electronics module consists of a power supply module 19a that converts the voltage supplied by the power supply servo 8 to 12V, which is then supplied to a set of current sources located in the control module 19c, powering the actuators in the form of 14 LEDs (from the ultraviolet to near-infrared wavelength range, i.e. 370 nm - 940 nm) 24. The control is based on the principle of setting the currents for a given channel (for a given LED) and switching this path via the local MCU (*microcontroller unit*) located in the HSI 19b control module block. All components are connected via vertical connectors, over which control and power signals are transmitted between the boards.

In this embodiment, the local MCU is a computer made on a single substrate, i.e. its entire layout is made as a single chip in which there is a CPU, memory, support for peripherals and data buses like USB, UART, I2C and other components.

In this embodiment, the HSI Handle 4 informs the user of its status during operation. For this purpose, two LEDs - the (e.g. green) indication LED 30 and the (e.g. red) information LED 31 (located in the upper part of the handle housing 18) keep you informed about the status of the device.

The readiness of the unit is signalled by the slow flashing of the green indicator LED 30. The image gathering process is signalled by the continuous light of the green indicator LED 30. Once the data has been stored correctly, the device switches back to standby. This state will be indicated by the signal diode 30 by its slow flashing. Error information is provided in the form of the red light of information LED 31.

At the same time, two main types of errors are foreseen:
- Continuous red light - contact the support team - manufacturer's service.
- If the red LED flashes, try the shooting procedure again. If the problem recurs, contact the support team.

Whereas a diagram of the laser hair removal head 3 is shown in fig. 3, on which 32 denotes laser slide; 33 denotes laser diode stacks of three wavelengths; 34 denotes laser diode stack mount 33; 35 denotes photodiode mount; 36 denotes cooling cell; 37 denotes cooling cell temperature sensor; 38 denotes cooling tip; 39 denotes mounting core; 40 denotes vibrating mechanism; 41 denotes heat receiver with input and output for cooling wires; 42 denotes humidity sensor; 43 denotes lower part of laser hair removal head housing; 44 denotes lower part of wire opening; 45 denotes upper part of wire opening; 46 denotes upper part of laser hair removal head housing; 47 denotes laser diode stack temperature sensor; 48 denotes hair removal head control module; 49 denotes waveguide; 50 denotes heat sinks; O denotes exit hole.

In this embodiment, the laser diode stack 33 comprises a stack of 1 diode 33a, a stack of 2 diodes 33b and a stack of 3 diodes 33c for three wavelengths, i.e. 808 nm (Vsilk 2-1200-808, 1200W), 1064 nm (Vsilk 2-1000-1060, 1000W), 755 nm (Vsilk 2-900-760, 900W) from Focus Light Technologies Inc. 33 and 50, while the cooling cell 36 is a Peltier cell.

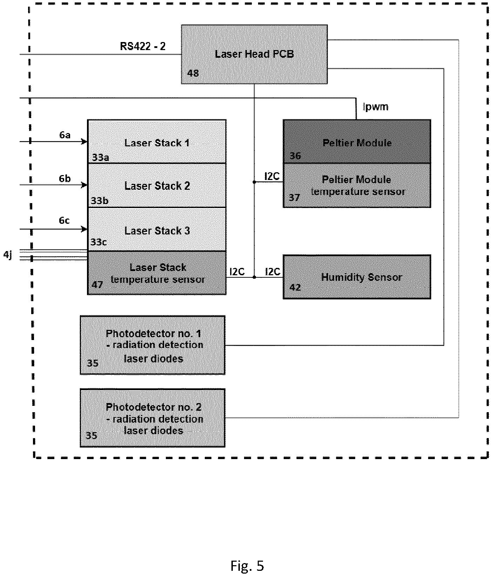

A block diagram of an example epilation head 3 is shown in fig. 5, wherein 4j denotes the cooling conductors fed to the base receiving heat from the laser stacks, 6a denotes the laser diode stack controller 1, 6b denotes the laser diode stack controller 2, 6c denotes the laser diode stack controller 3, 33a denotes the laser diode stack 1, 33b denotes laser diode stack 2, 33c denotes laser diode stack 3, 35 denotes photodiodes, 36 denotes cooling cell, 37 denotes cooling cell temperature sensor, 47 denotes laser diode stack temperature sensor, 48 denotes epilation head control module.

The head control module 48 continuously monitors and supervises the status of the epilation head 3 by cyclically checking the basic parameters with the help of the corresponding sensors, i.e. the humidity (via humidity sensor 42), the temperature of the laser diode stack (via sensor 47) as well as the temperature status of the Peltier cells (via sensor 42) and the photodetection of the operation of the photodiodes 35.

Whereby, said epilation head 3 constitutes the final implementing element of the device according to the invention, which has direct contact with the skin of the patients undergoing the epilation treatment.

In this embodiment, laser diode stacks 33a-33c are controlled by a signal from three driver 6 - power supplies FL-LPS-0115 from Focus Light Technologies Inc. (one power supply for each of the stacks). These, in turn, are driven directly from the central processing unit 11 (via Ready lines 1..3 activating signal outputs to laser stacks 33) and from a special electronics module called the power supply controller 8. It is this component that, after receiving the appropriate epilation parameters, drives the three PWM signals of the three aforementioned controllers 6. These, in turn, provide adequate power to the laser diode stacks 33 emitting radiation with the parameters desired by the user.

The power supply controller 8 has another additional function. Namely, it controls the Peltier cell 36 - cooling the epilated area of the patient's body through contact with a metal component in the form of a frame surrounding the exit aperture 38 of the laser 33 stacks, providing comfort to the client by taking away some of the heat from the conversion of the light energy supplied to the skin, which is converted into heat energy through the absorption of melanin and other compounds contained in the skin, locally increasing the temperature of the treatment area.

Each of the temperature and humidity sensors 37, 47, 42 used communicates with the head controller 48 via the I2C bus.

In this embodiment, optical sensors in the form of two photoelements 35 are used in the epilation head 3 to detect radiation. This is to increase patient safety in unforeseen situations due to the high optical power emitted by the laser diode stacks 33. Photoelements are used to confirm that a given laser diode stack is indeed emitting radiation. In addition, in the event of any malfunctions in the 33 laser stack, they allow a quick diagnosis of a possible fault in the laser emitters.

### Example 2.

### System according to the invention

A network system for epilation devices according to the invention is provided with one or more devices according to the invention. In this embodiment, the system is equipped with 10 devices according to example 1, a database and a cloud server.

Whereby, said database comprises skin colour data, hair colour data, skin photos, hair photos, photos of epilation treatments carried out, skin colour and hair classification, epilation head parameters matched to skin colour and hair colour, and a cloud server is configured to select laser epilation head setting parameters (configuration) based on skin colour and hair colour by a method according to the invention comprising:
a) Transmission of the request to measure the patient's skin and hair parameters to the central unit 11;
b) Initiation by the central unit 11 of the measurement of the patient's body temperature by sending a request to the HSI handle controller 19, which initiates the temperature measurement by means of a contactless temperature sensor 28 HSI handle 4;
c) Initiation by the central processing unit 11 of taking a series of images with the HSI camera 20 by sending a request to the HSI handle controller 19, where on each command issued by the central processing unit 11, the HSI handle controller 19 initiates the activation of at least one LED from the board 24 to illuminate the patient work area with the selected wavelength and to take an image with the HSI camera 20, and each of the images taken in the series in real time is evaluated by the central processing unit 11 for a focus and offset criterion;
d) Storing each of the images taken in the memory of the central unit 11 along with metadata including temperature and body humidity;
e) Transfer of stored images with metadata as input to the neural network implemented in the CPU memory 11;
f) Classification by neural network of the patient's skin and hair colour;
g) Transmission of the classification determined in step f) to a reinforced learning network which, on the basis of the input data, generates the optimal setting for the epilation head (3) in the form of a vector of parameters containing for each wavelength: laser intensity levels, pulse duration per period, operating frequency, length of operation of the machine at the given parameters, the said input data being at least the classification data from step f).

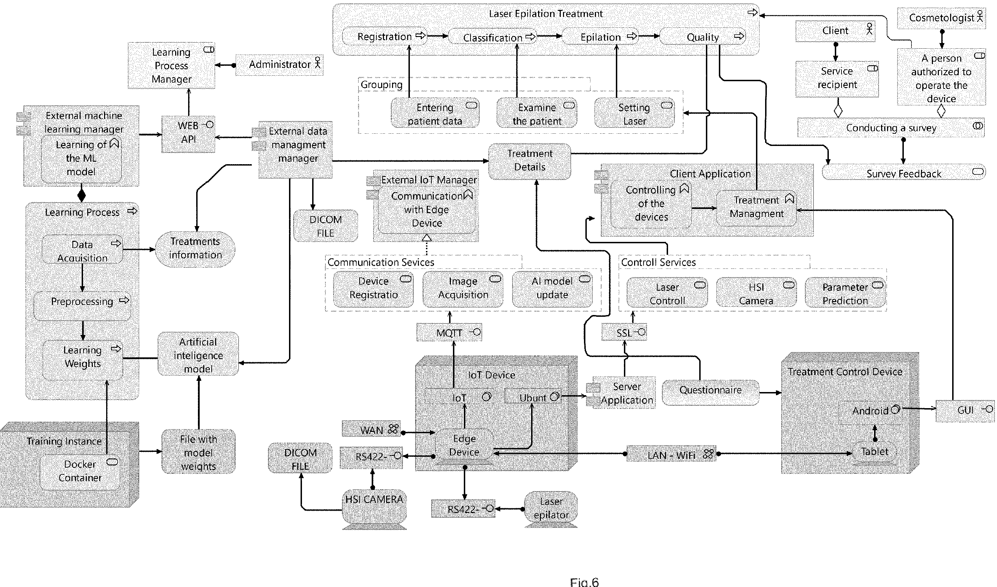

With the system of the invention, learning from newly acquired data is possible. In this embodiment, the cloud architecture has been implemented on an Amazon Web Services server service, along with Edge Computing appliances and an administration layer, as shown in fig. 6 on which the different layers of the system are marked.

### Example 3.

The method according to the invention comprises the following steps:
a) Transmission of the request to measure the patient's skin and hair parameters to the central unit 11;
b) Initiation by the central unit 11 of the measurement of the patient's body temperature by sending a request to the HSI handle controller 19, which initiates the temperature measurement by means of the contactless temperature sensor 28 of the HSI handle 4;
c) Initiation by the central processing unit 11 of taking a series of images with the HSI camera 20 by sending a request to the HSI handle controller 19, where on each command issued by the central processing unit 11, the HSI handle controller 19 initiates the activation of at least one LED from the board 24 to illuminate the patient work area with the selected wavelength and to take an image with the HSI camera 20, and each of the images taken in the series in real time is evaluated by the central processing unit 11 for a focus and offset criterion;
d) Storing each of the images taken in the memory of the central unit 11 along with metadata including temperature and body humidity;
e) Transfer of stored images with metadata as input to the neural network implemented in the CPU memory 11;
f) Classification by neural network of the patient's skin and hair colour;
g) transmission of the classification determined in step f) to a reinforced learning network which, on the basis of the input data, generates the optimal setting for the epilation head (3) in the form of a vector of parameters containing for each wavelength: laser intensity levels, pulse duration per period, operating frequency, length of operation of the machine at the given parameters, the said input data being at least the classification data from step f).

Whereby, in this non-limiting embodiment, the method according to the invention is performed in the device according to example 1 connected to the system according to example 2. However, a request to measure the patient's skin and hair parameters is sent to the central unit 11. In this embodiment, the method is initiated by a low state of the TTL 5V NVidia Trigger signal using button 29 located on the HSI 18 handle housing outline. The fact that button 29 is pressed. sends a user request to the central unit 11 with the command to start taking measurements of the patient's skin and hair parameters and adjusting the setting of the epilation head 3 accordingly.

The next step is that the central unit 11 initiates the measurement of the patient's body temperature by sending a request to the HSI handle controller 19, which initiates the temperature measurement using the contactless temperature sensor 28 of the HSI handle 4. In this embodiment, the contactless temperature sensor 28 is a Melexis MLX90632 temperature sensor placed at the base of the HSI 20 camera in the HSI 18 handle housing.

The next step is for the central unit 11 to initiate the taking of a series of images with the HSI camera 20 by sending a request to the HSI handle controller 19, starting an exchange of data with it, while the aforementioned controller 19, by means of pre-selected lighting parameters for each LED, controls the LEDs by lighting them for a specific time, using in addition a PID algorithm that makes use of the feedback of the photodiode located on the illumination board 24.

For each command issued by the central unit 11, the HSI 19 handle controller responds with a report of the execution of the corresponding command using the RS422 protocol. In parallel, while a specific LED is switched on and thus illuminating the patient's skin working area with a specific wavelength, an image is taken with a camera 20 with a resolution of 2592 (H) x 1944 (V).

The following wavelengths are used in this example of taking the aforementioned series of images: 370 nm, 390 nm, 450 nm, 500 nm, 520 nm, 540 nm, 590 nm, 623 nm, 660 nm, 740 nm, 810 nm, 850 nm, 940 nm and a broadband diode with wavelengths of 450 nm, 750 nm and 1050 nm.

At the same time, each of the images taken on the fly is immediately subjected to an initial assessment of its suitability in terms of the sharpness criterion and offset by the central unit 11. If the photo does not meet the quality requirements, another attempt is made. In this embodiment, one series of images is a set of 14 images.

The next step is to store each of the images taken in the central unit 11's memory in 14-channel format, with metadata added in the form of body temperature and body humidity. Such data goes to the input of the neural network implemented in the central processing unit 11, which is learned for the given task and, consequently, its weights are frozen. The output of the network gives us the probability with which a given skin and hair colour appears in the image.

This information is then used in an algorithm running on the central processing unit 11 to propose a setting for the epilation head 3; i.e. the established classification is passed to the reinforced learning network implemented on the central processing unit 11, which, on the basis of the input data, generates an optimal setting for the epilation head in the form of a parameter vector containing for each wavelength: laser intensity levels, pulse duration per period, operating frequency, length of operation of the machine at the given parameters.

In this embodiment, the input data includes a skin and hair colour classification determined by the measurements taken and metadata including the part of the body part being epilated, gender, age, personal data of the patient in question.

Furthermore, in this embodiment, the proposal for the optimal setting of the epilation head 3 is displayed in real time on the display screen 5 (e.g. tablet, monitor, etc.), to the person performing the treatment, who, according to his/her expert judgement, accepts it or changes it if the proposal is not in accordance with his/her knowledge and experience. The entire cycle, including running the classification through a machine learning model with a laser setting proposal, takes up to 5 seconds for the complete data set obtained, without repetition.

### Example 4.

The method as in example 3, except that it includes a step f '), in which the photo is segmented using a neural network of hair follicles, the number of follicles is counted and the number of hairs visible in the photo is sent to the Reinforcement Learning network as one of the metadata needed to choose its parameters. Furthermore, in this example implementation, the method according to the invention comprises the step of training a neural network for skin and hair colour classification and a reinforcement learning network by uploading the results obtained in steps f) and g) to a cloud server, where training instances of the network are created on the basis of the newly added data in combination with the data contained in the database and an additional evaluation of the images in terms of hair density and skin condition of the client is carried out, and the obtained results of the analysis are sent to the memory of the central unit (11) in order to train the networks stored therein to perform steps f) and g).

The result of the image classification obtained in step f), is the assignment of skin colour and hair colour per downloaded image. The network is apprenticed in a supervised manner - the images are marked by a dermatologist. The images and classification results are downloaded to the cloud, where the neural network is trained to improve classification. Training is carried out on multiple instances created on the server side using mlflow, docera and the Python language. Once the specialist has recognised the improved classification capabilities of the network, its weights are sent to the devices according to the invention distributed and uploaded to the existing network on them. The neural network for skin and hair classification is a block of the larger Reinforcement Learning network. This network is intended to select the parameters of the laser hair removal treatments in step (g) of the method according to the invention by means of received input data such as - the patient's skin and hair colour, the patient's temperature and humidity, the density and structure of the roots and hairs, the general condition of the skin. This data is received from the HSI 20 camera and the sensors in the HSI 4 handle. The input data are also the starting parameters set by the dermatologist, appropriate to the laser being created. By assessing the condition of the skin, this network slightly alters the laser hair removal parameters - with the possibility of changing up to impassable limits of the laser parameters - so as to improve (minimise) hairs in the treatment area. Collection of data from the patient of the area to be epilated, takes place at each visit to the facility, so that it is possible to assess the skin in time and the function of the target which minimises the time required to achieve the desired result, hairlessness. The model, parameters, patient data such as images and metadata are stored in the database of the system according to the invention.

In this embodiment, in order to train the network to classify skin and hair colour, images in DICOM format are uploaded to a server and stored on the cloud server of the system according to the invention. The downloaded 14-channel RGB images (3 channels for red, green and blue x 14 images for each wavelength - 44 channels), in DICOM format, go into the pre-processing block. This block processes the pixels using successively: the SHARPEN (sharpening) algorithm in the form of matrix multiplication in a spliced manner of the image data from an appropriately sized kernel, resulting in a sharpened image. The images are then processed by the CLAHE (Contrast Limited Adaptive Histogram Equalization) algorithm, which adaptively improves the quality of the images in the form of removing saturation on the dark side as well as the light side (0.255 for each channel). Then, the 14-channel RGB data, processed in this way, goes to a centring algorithm which compares between channel offsets and automatically shifts, globally, each image relative to the selected reference image, by the appropriate number of pixels, so that the skin elements for each channel overlap as much as possible. Once the transformation between channels has been carried out in this way, they are combined by inter-channel averaging, between the RGB bit information for each image. This produces an RGB image that has hyperspectral information in the image. Each colour channel from the image (R, G, B) can be processed individually or all of them simultaneously. The data processed in this way consists of a series of RGB images or a series of greyscale maps for each channel.

As part of the training of the network for skin and hair classification, this image is taken from a database in which images from all epilation treatments are stored. These images as a series are fed into the neural network model. The output of the neural network is divided into two linear layers, related to the prediction of skin colour and hair colour. A SoftMax function is used for each of the outputs, as well as a cross entropy function to calculate a loss value comparing the labelling of the images with the network predictions. This loss is then added up and used for the back propagation algorithm. Training is stopped for test set loss values at an acceptable level (85% AUC ROC),
when a suitable value is obtained for the ability of the network to classify skin and hair colours. For prediction purposes in the solution running in the treatment room, the model is saved to the cloud. Operating as an Edge type, the device downloads the latest available version of the model to its local memory. This time, the images are taken directly from the hyperspectral camera 20 and processed in the same way as before only on local devices (in their central units 11), instead of in the cloud as before with the network training process. The network output classifies skin and hair colour. This data is displayed on display 5, for inspection by those in the practice.

The second algorithm used in the solution according to the invention is the hair follicle segmentation algorithm. As in the previous model, the training process is carried out by using the collected customer images in the database space. These are uploaded to the pre-processing layer. Then, they are sent to the input of the machine learning network. Each photo is annotated with the location of hair follicles marked by hand, by a person with the relevant expertise. Based on these markings, binary maps are produced from the RGB images, which are compared with the neural network's prediction samples also in the form of a binary map typed by the network which effectively means determining a class for each pixel in the image of type is or is not a hair root. In this way, a loss function is determined that changes the weights of the network during learning, using a back-propagation algorithm. In this manner the learning process is carried out until the effectiveness of the network reaches above 85% AUC ROC. The prediction process takes place as in the previous algorithm, i.e. the images are taken from a local device (i.e. the device according to the invention), the HSI 20 camera, which sends them to the pre-processing layer. Once processed, this image goes to the network, which creates predictions in the form of a binary map of the location of hair follicles. In addition to the binary map, a hair follicle counting algorithm is performed, which finds the areas of the binary map that represent hair follicles. This determines the density of hair on a given area of the body.

Parallel to the segmentation algorithm is the hair follicle detection algorithm, which can be used interchangeably with that for segmentation. Its block diagram is shown in Figure 12. It differs from the previous algorithm only in that training does not take place for every pixel, but rather for the areas marked by the marker. This is the most classic type of network for image recognition using convolution networks. The algorithm learns to detect larger elements from an appropriately labelled image added by a qualified cosmetologist or dermatologist. On this basis, the number of elements labelled in the image by the network can be counted in the prediction phase, which should effectively give the hair density.

In contrast, in the training process, the reinforcement learning network uses a GAN-type network architecture in a reinforcement learning configuration. It is made up of two neural networks. The first network is responsible for generating the laser settings for epilation, in the form of a parameter vector containing for each wavelength: laser intensity levels, pulse duration per period and operating frequency. It also includes the length of time the machine has been running on the given parameters. This network chooses the aforementioned parameters based on skin and hair colour classification and meta data such as the part of the body part being epilated, gender, age and many other personal details of the patient in question.

The second network in this architecture is used to type whether the data generated by the first network is correct or not, or in GAN nomenclature 'False' or 'True'. Learning works on the principle of the first network generating data and giving the true data alternately (preferably at random) and the second network choosing whether the data shown is false or true. Obtaining a probability score of 0.5 for all data shown means that the true data is indistinguishable from the generated data. In addition, the reinforcement learning mode, i.e. in the manner of RL learning (reinforced learning, from the Reinforcement Learning), on the principle of rewarding the network for correct results and punishing undesirable results, using a transformed Bellman formula.

Learning takes place in two stages. In the first stage, the generator in the GAN chooses the parameters based on this meta-data, with the discriminator network frozen. This means that the generator becomes better in theory than the discriminator rating, which is designed to assess whether the generator has chosen good parameters. This assessment is based on a comparison with real laser settings selected on the basis of a visual assessment and data from a qualified employee. A loss function is used between these data to bring the actual and generated parameter settings closer together. In the second step, the generator network is frozen and the discriminator network learns to better recognise whether the settings at its input are true or false - the settings are randomised between the generator data set and the true laser settings. This allows the discriminator to learn which data are false and which are true. The cycle is repeated until the discriminator cannot distinguish between the true data and that generated by the generator, which means that the generator has learnt the space in which the skilled true-set typist moves and successfully handles the choosing. In the next step, the network from the training phase is transferred to the prediction phase on local devices, as in the previous way using EDGE-type devices, downloading images with a Pre-processing layer and classification on skin and hair colour devices. This time, however, this data, along with the customer's data, goes to the network for choosing laser setting parameters. This network learns online, which means learning as you go through the treatment process. The network is taught using the TD-lambda method of reinforcement learning. As the maximum number of laser hair removal treatments is 8, for any part of the body, the aim of this network is to minimise the number of treatments needed. This is done through a segmentation and hair follicle detection algorithm from the HSI images acquired, through which the amount of hair reduction is counted every time a treatment is performed. For the associated laser settings selected by the network with a given degree of hair reduction, the network is rewarded or penalised accordingly, allowing the network to create an optimal strategy in the context of the target. In parallel or alternatively, the input to the network can be a hair reduction assessment by a qualified professional, who enters the relevant values into the system for the client in question, from where the network retrieves the relevant data needed to learn the hidden layers.

### Example 5.

Method as in example 4, except that the series of images taken in step c) are 3 images.

### Example 6.

Method as in example 4, except that the series of images taken in step c) is 10 images.

### Example 7.

Method as in example 4, except that the series of images taken in step c) is 50 images.

### Example 8.

Method as in example 4, except that the series of images taken in step c) is 150 images.

### Example 9.

Method as in example 4, except that the series of images taken in step c) is 500 images.

## Claims

1. A method for selecting the setting parameters of a laser hair removal head (3) based on skin and hair colour, **characterised in that** it comprises the following steps:
a) Transmission of the request to measure the patient's skin and hair parameters to the central unit 11;
b) Initiation by the central unit (11) of the measurement of the patient's body temperature by sending a request to the HSI handle controller (19), which initiates the temperature measurement by means of the contactless temperature sensor (28) of the HSI handle (4);
c) Initiation by the central processing unit (11) of taking a series of images with the HSI camera (20) by sending a request to the HSI handle controller (19), where on each command issued by the central processing unit (11), the HSI handle controller (19) initiates the activation of at least one LED from the board (24) to illuminate the patient work area with the selected wavelength and to take an image with the HSI camera (20), and each of the images taken in the series in real time is evaluated by the central processing unit (11) for a focus and offset criterion;
d) Storing each of the images taken in the memory of the central unit (11) along with metadata including temperature and body humidity;
e) Transfer of stored images with metadata as input to the neural network implemented in the CPU memory (11);
f) Classification by neural network of the patient's skin and hair colour;
g) Transmission of the classification determined in step f) to the reinforcement learning network (reinforcement learning), which, on the basis of input data, generates the optimum setting for the epilation head (3) in the form of a parameter vector containing for each wavelength: laser intensity levels, pulse duration per period, operating frequency, length of time the machine operates at the given parameters, the said input data being at least the classification data from step (f).

2. The method according to claim 1, **characterised in that** in step b) wavelengths in the range from 370 nm to 1050 nm are used to take a series of pictures by the HSI camera (20).

3. The method according to claim 1 or 2, **characterised in that** that the series of images taken in step c) consists of at least 3 images, preferably 5 images, 10 images, 14 images, 50 images, 150 images, 500 images.

4. The method according to any of the preceding claims 1 to 3, **characterised in that** it comprises the step of validating the selected setting of the epilation head (3) by presenting the selected setting to a specialist performing the epilation treatment with the option of manual adjustment.

5. The method according to any of the preceding claims 1 to 4, **characterised in that** in step (g) the input data used are the data obtained from step (f) in combination with at least one parameter selected from a group of metadata comprising: part of the body part epilated, gender, age, personal data of the patient in question, information on pregnancy, information on breastfeeding, information on autoimmune diseases, data on medications taken, information on cosmetics used, information on herbal remedies taken, information on visits to a solarium in the last 4-6 weeks, date of last epilation, date of last peeling and type of peeling, tendency of the skin to hyperpigmentation, occurrence of dermatological skin lesions, occurrence of Reticularis, history of photodermatoses, history of past or ongoing viral/fungal/bacterial infections, history of epilepsy, history of diabetes mellitus, presence of pacemaker, use of insulin pump, history of scarring, HPV infection, history of eczema, difficulties with wound healing, use of steroid medication, complexion type, presence of ingrown hairs, presence of folliculitis, presence of hormonal disorders, any tattoos, any permanent make-up, any implants or combination of the above.

6. The method according to any of the preceding claims 1 to 5, **characterised in that** it comprises a step of training a neural network for skin and hair colour classification and a reinforcement learning network by uploading the results obtained in steps (f) and (g) to a cloud server, in which training instances of the network are created on the basis of the newly added data in combination with the data contained in the database and an additional evaluation of the images in terms of hair density and skin condition of the client is carried out, and the analysis results obtained are transferred to the memory of the central unit (11) in order to train the networks stored therein to perform steps f) and g).

7. The method according to any of the preceding claims 1 to 6, **characterised in that** step f) is followed by step f '), wherein the photo is segmented by a neural network of hair follicles, the number of follicles is counted and the number of hairs visible in the photo is sent to the reinforcement learning network.

8. An epilation device comprising a body, a central unit, a display, a cooling module, power supplies and a laser epilation head, **characterised in that:**
it is equipped with an HSI handle (4), which consists of a housing (18), inside of which a hygroscopic camera (20) with lens (21), a diode board (24), and an HSI handle controller (19) are mounted, while on the bottom of the housing (18), below the HSI camera (19), a contactless temperature sensor (28) is placed;
the laser epilation head (3) is equipped with a three-wavelength laser diode stack (33) and a epilation handpiece controller module (48) for setting treatment parameters based on data from the central unit (11);
the central unit (11) comprises a processor and a memory, with Edge Computing architecture implemented, containing instructions for performing the selection of the setting parameters of the laser hair removal head (3) based on skin and hair colour by the method specified in any of the preceding claims 1 to 7.

9. The epilation device according to claim 8, **characterised in that** the housing of the HSI handle (4) is provided with an indication diode (30) and an information diode (31).

10. The epilation device according to claim 8 or 9, **characterised in that** the three wavelengths of the laser diode stack (33) include wavelengths of 808±5 nm, 1064±5 nm and 755±5 nm, respectively.

11. The epilation device according to any of the preceding claims 8 to 10, **characterised in that** the handle of the HSI (4) is housed in a lockable pocket of the body (2).

12. The epilation device according to any of the preceding claims 1 to 11, **characterised in that** each diode of the laser diode stack (33) is independently controlled by a dedicated diode stack controller (6) of the laser epilation head (3).

13. An epilation device network system having at least one epilation device, a cloud server and a database, **characterised in that** said at least one epilation device is a device as defined in any of the preceding claims 8 to 12, the database comprises skin colour data, hair colour data, skin photos, hair photos, photos of epilation treatments performed, skin colour and hair classification, parameters of the epilation head (3) matched to the skin colour and hair colour, and the cloud server is configured to select the setting parameters of the laser epilation head (3) based on the skin colour and hair colour by the method defined in any of the preceding claims 1 to 7.
